(19) Europäisches Patentamt
European Patent Office
Office européen des brevets

(11) Numéro de publication: **0 067 769**
**A1**

(12) # DEMANDE DE BREVET EUROPEEN

(21) Numéro de dépôt: 82401058.1

(22) Date de dépôt: 11.06.82

(51) Int. Cl.[3]: C 07 D 307/91
A 61 K 31/34

(30) Priorité: 12.06.81 FR 8111672

(43) Date de publication de la demande: 22.12.82 Bulletin 82/51

(84) Etats contractants désignés: AT BE CH DE FR GB IT LI LU NL SE

(71) Demandeur: RIOM LABORATOIRES- C.E.R.M. (Société Anonyme)
Route de Marsat
F-63203 Riom Cedex(FR)

(72) Inventeur: Combourieu, Michel
6, rue du Mont Mouchet
F-15000 Aurillac(FR)

(72) Inventeur: Laigle, Jean-Claude
Résidence Versailles, St 25 Bt C
F-15000 Aurillac(FR)

(72) Inventeur: Busch, Norbert
Le Bouquet Loubeyrat
F-63410 Manzat(FR)

(74) Mandataire: Therond, Gérard Raymond et al,
RL-CERM Route de Marsat
F-63203 Riom Cedex(FR)

(54) **1,2,3,4,4a,9b-hexahydro-4a-pipérazinylméthyl-4-dibenzofuranones ou 4-dibenzofuranols substitués, procédé de synthèse et application en thérapeutique.**

(57) La présente invention concerne des composés de formule:

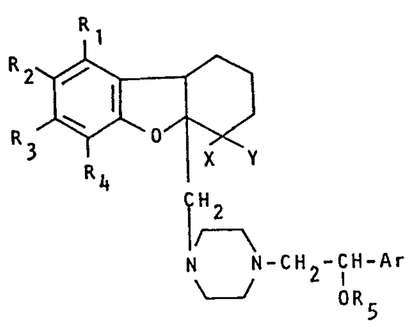

et leurs sels pharmaceutiquement acceptables, dans laquelle $R_1$, $R_2$, $R_3$ et $R_4$ sont identiques ou différents et représentent un atome d'hydrogène, un atome d'halogène, un radical alkyle ou alcoxy inférieur ou le radical trifluorométhyle; $R_5$ représente l'hydrogène ou un radical alkyl inférieur; X représente un atome d'hydrogène et Y le radical hydroxy, ou bien X et Y représentent ensemble un atome d'oxygène, et Ar représente un reste aromatique éventuellement substitué.

Application comme anti-bronchoconstricteur.

EP 0 067 769 A1

0067769



1,2,3,4,4a,9b-hexahydro-4a-pipérazinylméthyl-4-dibenzofuranones ou 4-dibenzofuranols substitués, procédé de synthèse et application en thérapeutique

La présente invention concerne des 1,2,3,4,4a,9b-hexahydro-4a-pipérazinylméthyl-4-dibenzofuranones ou 4-dibenzofuranols substitués, leurs procédés de synthèse et les préparations pharmaceutiques les contenant.

L'invention concerne plus particulièrement des composés représentés par la formule générale I suivante :

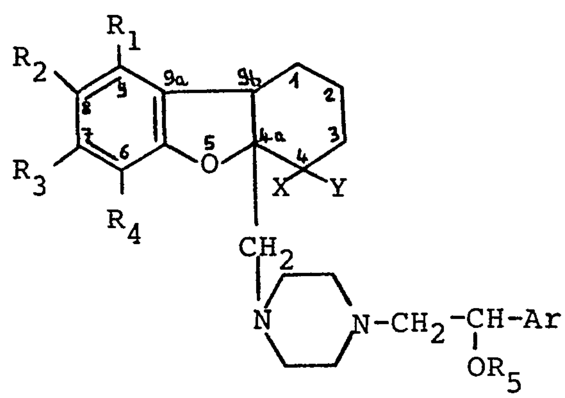

I

et leurs sels pharmaceutiquement acceptables dans laquelle $R_1$, $R_2$, $R_3$ et $R_4$ sont identiques ou différents et représentent un atome d'hydrogène, un atome d'halogène, un radical alkyle ou alcoxy inférieur ou le radical trifluorométhyle ;

$R_5$ représente l'hydrogène ou un radical alkyle inférieur; X représente un atome d'hydrogène et Y représente le radical hydroxy, ou bien X et Y représentent ensemble un atome d'oxygène, et Ar représente un reste aromatique éventuellement substitué.

Un radical alkyle inférieur, tel qu'utilisé dans les définitions de $R_1$ à $R_4$ et $R_5$, désigne un radical alkyle, linéaire ou ramifié ayant 1 à 6 atomes de carbone et de préférence 1 à 4 atomes de carbone tel que les radicaux méthyle, éthyle, propyle, n-butyle, sec-butyle et ter-butyle. Le reste alkyle du radical alcoxy a la même signification.

Halogène signifie fluor, chlore, brome ou iode dont le fluor et le chlore représentent la signification préférée.

Le reste aromatique tel qu'indiqué dans la définition de Ar est de préférence le radical phényle ou pyridyle, éventuellement substitué par un radical alkyle de 1 à 4 atomes de carbone, un radical alcoxy de 1 à 4 atomes de carbone, ou un halogène. Selon les réalisations préférées les restes aromatiques sont : phényle, phényle substitué par un alkyle, 3-pyridyle et 4-méthyl-3-pyridyle.

De nombreuses publications ont été effectuées sur des hexahydrodibenzofurannes, mais en aucun cas les substitutions envisagées dans les molécules décrites ne correspondent aux substitutions particulières des composés de l'invention. A titre d'exemple pouvant être considéré comme le plus proche de la présente invention, on peut citer le brevet US 3 496 181 décrivant un 1,2,3,4,4a,9b-hexahydro-4a-(4-méthyl 1-pipérazinyl)-8-dibenzofuranol, non pour son application en tant que

médicament, mais comme intermédiaire donnant par hydrogénation catalytique des composés inhibiteurs de la pseudocholinesterase.

Les composés de formule I peuvent être préparés par des méthodes actuellement utilisées ou décrites dans la littérature. On peut par exemple préparer les composés de formule I par condensation d'un composé de formule II :

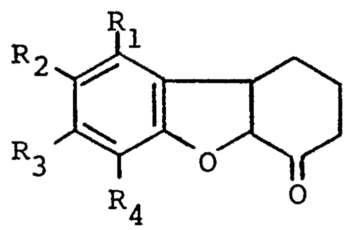

II

dans laquelle $R_1$, $R_2$, $R_3$ et $R_4$ ont les significations précédemment mentionnées, avec un composé de formule III:

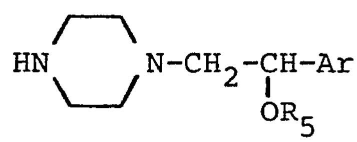

III

dans laquelle $R_5$ et Ar ont les significations précédemment mentionnées, en présence de formaldéhyde ou de paraformaldéhyde. Cette condensation, qui est connue sous le nom de réaction de Mannich, s'effectue de préférence dans l'éthanol absolu, en présence d'acide chlorhydrique, avec un excès de paraformaldéhyde.

La réaction de condensation conduit à un composé de formule I dans laquelle X et Y représentent ensemble l'oxygène.

Pour obtenir les composés de formule I, dans laquelle X représente l'hydrogène et Y le radical hydroxy, on effectue une réduction en plus de la réaction de Mannich précédente, par exemple au moyen d'un hydrure métallique complexe tel que le borohydrure de sodium.

Les 1,2,3,4,4a,9b hexahydro-4-dibenzofuranones de formule II utilisées comme matières premières peuvent elles-mêmes être préparées, par exemple, à partir d'un 2-(cyclohexényl-2) phénol traité par un peracide organique pour donner une 1,2,3,4,4a,9b hexahydro-4-dibenzofuranol dont la fonction hydroxy est ensuite oxydée, par exemple au moyen du chlorochromate de pyridinium.

Les sels pharmaceutiquement acceptables des composés de formule I sont obtenus en faisant réagir un composé I avec un acide organique ou inorganique convenable tels que HCl, HBr, les acides phosphorique,acétique, maléique, fumarique, tartrique, citrique, etc...

Les composés de formule I contiennent des carbones asymétriques de sorte que des stéréoisomères de la formule I sont possibles. Ces stéréoisomères (sous forme de racémique ou sous forme d'énantiomères séparés) font également partie des composés de l'invention. Lesdits stéréoisomères peuvent être séparés du mélange par des techniques conventionnelles de séparation et/ou résolution.

Les composés selon l'invention et leurs sels pharmaceutiquement acceptables sont utiles en tant que principes actifs pharmaceutiques,notamment en raison de leurs remarquables propriétés antibronchoconstrictrices.

Associés aux excipients pharmaceutiques habituels, les composés de l'invention peuvent être administrés par voie enthérale ou parenthérale, de préférence à une dose journalière comprise entre 0,5 et 25 mg par kg de poids corporel. La dose préférée en thérapeutique humaine est comprise entre 200 et 1000 mg par voie orale.

L'activité antibronchoconstrictrice des composés de l'invention a été évaluée chez le cobaye selon la technique de KONZETT et ROSSLER.
Après anesthésie au carbamate d'éthyle et trachéotomie, les animaux sont ventilés artificiellement à volume constant et la pression intertrachéale est enregistrée à chaque instant, ce qui permet la mesure indirecte de la résistance totale pulmonaire. L'histamine (5 $\mu g.kg^{-1}$ I.V.) est administrée avant et à différents intervalles de temps après administration des composés de l'invention par voie I.V.
On évalue l'activité du produit étudié en comparant les amplitudes des bronchospasmes avant et après traitement et en calculant le pourcentage de variation. On a également noté la durée d'action des composés étudiés.
Les résultats enregistrés sont rapportés dans le tableau I ci-après.
Dans ce tableau, on a également reporté une estimation de toxicité aiguë DL 50 effectuée par voie orale chez la souris.

TABLEAU I

| COMPOSE N° (voir tableau II des exemples) | DOSE (en $mg.kg^{-1}$ I.V.) | POURCENTAGE D'INHIBITION | DUREE (en min.) | ESTIMATION DL 50 ($mg.kg^{-1}$) |
|---|---|---|---|---|
| 1 | 5<br>1 | 100 ± 0<br>87,7 ± 6,6 | > 60<br>> 60 | 900 |
| 2 | 5 | 95 ± 3,1 | > 60 | 900 |
| 3 | 5 | 97,5 ± 2,5 | > 45 | 320 |
| 5 | 5 | 80,2 ± 9,9 | > 45 | 900 |
| 6 | 5 | 48,2 ± 9,2 | 45 | 900 |
| 8 | 1 | 95,5 ± 4,6 | > 60 | 480 |

On a en outre répété l'expérience sur le bronchospasme mais en remplaçant l'histamine par la sérotonine (20 µg.kg$^{-1}$ I.V.) : pour le composé n° 1 administré à 5 mg.kg$^{-1}$ I.V., on a alors observé une inhibition de 87,6 % pendant 60 minutes.

Ces résultats montrent que les composés de l'invention inhibent de façon intense et durable le bronchospasme histaminique et sérotoninergique. On peut donc envisager l'application de ces composés en thérapeutique humaine comme antibronchoconstricteur.

La préparation des composés de l'invention est illustrée plus en détail par référence aux exemples suivants.

## Exemple 1

1,2,3,4,4a,9b hexahydro-4a-(1-(4-(2-éthoxy-2-phényl)éthyl) pipérazinyl) méthyl-4-dibenzofuranone

Dans un réacteur contenant 200 ml d'éthanol absolu, on a introduit 12 ml d'acide chlorhydrique concentré, 25 g (0,133 M)de 1,2,3,4,4a,9b hexahydro 4-dibenzofuranone et 23,4 g (0,1 M) de (2-éthoxy 2-phényl) éthyl pipérazine.

Après dissolution, on a ajouté 7,98 g (0,266 M) de polyoxyméthylène et porté le mélange à reflux pendant 2 heures. Après avoir laissé le mélange revenir à environ 50°C, on a encore ajouté 4 g (0,133 M) de polyoxyméthylène et porté à nouveau le mélange à reflux pendant 2 heures. En fin de réaction, on a évaporé l'éthanol sous pression réduite, dissous le résidu dans l'eau et ajouté de la soude pour revenir en milieu basique.

On a extrait le produit du titre par le chlorure de méthylène, séché sur sulfate de sodium, évaporé le solvant et repris le résidu par de l'acétone anhydre.

En faisant passer un courant de gaz chlorhydrique sec, on a précipité le produit du titre sous forme de dichlorhydrate monohydraté.
Après recristallisation dans l'éthanol, on a obtenu 32,5 g de produit ayant pour point de fusion F = 182,7°C et pour analyse élémentaire :

| | C % | H % | N % |
|---|---|---|---|
| Calculé | 61,71 | 7,29 | 5,33 |
| Trouvé | 62,45 | 7,23 | 5,41 |

Exemple 2

1,2,3,4,4a,9b hexahydro-4a-(1-(4-(2-éthoxy-2-phényl) éthyl ) pipérazinyl) méthyl-4-dibenzofuranol
On a dissous 21,73 g (0,05 M) de 1,2,3,4,4a,9b hexahydro-4a-(1-(4-(2-éthoxy-2-phényl) éthyl) pipérazinyl) méthyl-4-dibenzofuranone dans 100 ml de méthanol, puis ajouté 1,97 g (0,05 M) de borohydrure de sodium et maintenu le mélange sous agitation à température ambiante pendant 2 heures. Après avoir évaporé le méthanol et ajouté de l'eau, on a extrait le produit formé à l'éther, séché sur sulfate de sodium, puis évaporé l'éther et repris le résidu par l'acétone et précipité le produit du titre sous forme de chlorhydrate en faisant passer dans la solution un courant d'acide chlorhydrique gazeux sec.
On a obtenu 15 g de produit du titre sous forme de dichlorhydrate monohydraté ayant pour point de fusion F = 238,6°C et pour analyse élémentaire :

| | C % | H % | N % |
|---|---|---|---|
| Calculé | 61,48 | 7,64 | 5,31 |
| Trouvé | 61,40 | 7,49 | 5,18 |

Exemple 3

1,2,3,4,4a,9b hexahydro-4a-(1-(4-(2-éthoxy-2-phényl) éthyl pipérazinyl) méthyl-8-fluoro-4-dibenzofuranol, 2 HCl

Dans une première étape, on a fait agir 20,6 g (0,1 M) de 1,2,3,4,4a,9b hexahydro-8-fluoro-4-dibenzofuranone sur 23,4 g de (2-éthoxy 2-phényl) éthyl pipérazine en suivant la méthode décrite en détail dans l'exemple 1, et on a obtenu 41,3 g de 1,2,3,4,4a,9b hexahydro-4a-(1-(4-(2-éthoxy-2-phényl) éthyl) pipérazinyl) méthyl-8-fluoro-4-dibenzofuranone dichlorhydraté.

Dans la deuxième étape, on a réduit 20 g (0,044 M) de cétone précédemment obtenue par 2 g (0,053 M) de borohydrure de sodium en appliquant le même processus que celui décrit dans l'exemple 2.

On a ainsi obtenu 21 g de composé du titre sous forme de dichlorhydrate ayant pour point de fusion F = 244,4°C et pour analyse élémentaire :

| | C % | H % | N % |
|---|---|---|---|
| Calculé | 61,48 | 7,07 | 5,31 |
| Trouvé | 60,89 | 7,22 | 5,35 |

Exemple 4

Divers autres composés de l'invention ont également été préparés selon les mêmes procédés que ceux décrits dans les exemples précédents.

Le tableau II indique les caractéristiques de ces composés.

TABLEAU II

| COMPOSE N° | $R_2$ | $R_4$ | 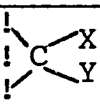 | $R_5$ | Ar | POINT DE FUSION (Sel) |
|---|---|---|---|---|---|---|
| 1 [Exemple 2] | H | H | >CHOH | $-C_2H_5$ | 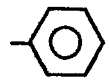 | 238,6°C ($2HCl$, $H_2O$) |
| 2 [Exemple 3] | F | H | >CHOH | $-C_2H_5$ | 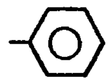 | 244,4°C (2 $HCl$) |
| 3 | H | $-OCH_3$ | >C=O | $-C_2H_5$ | 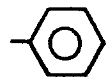 | 207,6°C (2 $HCl$) |
| 4 | H | $-OCH_3$ | >CHOH | $-C_2H_5$ | 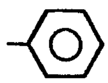 | 235,8°C (2 $HCl$) |
| 5 [Exemple 1] | H | H | >C=O | $-C_2H_5$ | 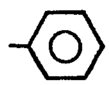 | 182,7°C ($2HCl$, $H_2O$) |
| 6 | $-OCH_3$ | H | >CHOH | $-C_2H_5$ | 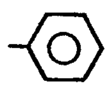 | 227,4°C (2 $HCl$) |
| 7 | H | H | >CHOH | $-CH_3$ | 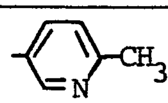 | 208°C (3 $HCl$) |
| 8 | H | H | >CHOH | $-CH_3$ | 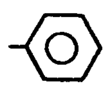 | 254,1°C (2 $HCl$) |
| 9 | H | H | >CHOH | H | 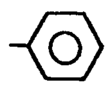 | 243,1°C (2 $HCl$) |

Les substituants $R_1$ et $R_3$ représentent l'hydrogène dans tous les composés précités de formule I.

## REVENDICATIONS

1. Composés répondant à la formule

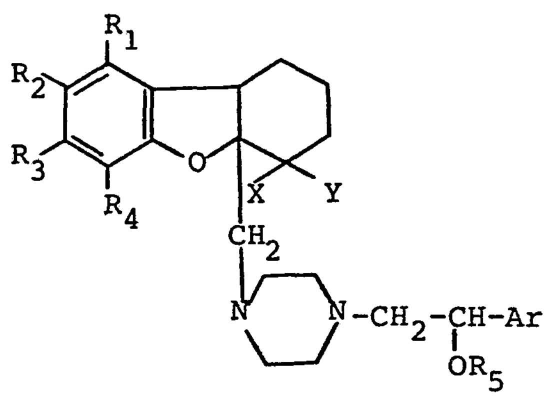

et leurs sels pharmaceutiquement acceptables, dans laquelle $R_1$, $R_2$, $R_3$ et $R_4$ sont identiques ou différents et représentent l'hydrogène, un halogène, un radical alkyle ou alcoxy inférieur, ou le radical trifluorométhyle ; $R_5$ représente l'hydrogène ou un radical alkyle inférieur ; X représente l'hydrogène et Y représente le radical hydroxy ou X et Y représentent ensemble un atome d'oxygène ; Ar représente un reste aromatique éventuellement substitué.

2. Composés selon la revendication 1 caractérisés en ce que X et Y représentent ensemble l'oxygène.

3. Composés selon la revendication 1 caractérisés en ce que X représente l'hydrogène et Y représente le radical hydroxy.

4. Composés selon les revendications 1,2,3 caractérisés en ce que $R_1$ et $R_3$ représentent l'hydrogène et $R_5$ représente l'hydrogène, le radical méthyle ou le radical éthyle.

5. Composés selon les revendications 1,2,3 caractérisés en ce que Ar représente le radical phényle, un radical phényle substitué, le radical pyridyle ou le radical 4-méthyl-pyridyl.

6. 1,2,3,4,4a,9b hexahydro-4a-(1-(4(2-alcoxy-2-phényl) éthyl) pipérazinyl) méthyl-4-dibenzofuranols et leurs sels pharmaceutiquement acceptables.

7. Procédé de préparation d'un composé de formule I tel que revendiqué dans la revendication 1 caractérisé en ce que le composé est préparé de façon connue en soit pour des composés analogues.

8. Procédé selon la revendication 7, caractérisé en ce qu'on fait réagir un composé de formule II avec un composé de formule III, ou son sel, en présence de formaldéhyde ou de paraformaldéhyde et qu'ensuite on réduit si nécessaire le composé obtenu, et/ou éventuellement on le transforme en sel pharmaceutiquement acceptable.

9. Composition pharmaceutique, utilisable en particulier pour le traitement des dyspnées, caractérisée en ce qu'elle contient, à titre de principe actif associé aux excipients pharmaceutiques habituels, au moins un composé selon l'une des revendications 1 à 6.

10. Composition pharmaceutique selon la revendication 9, possèdant des propriétés antibronchoconsctrictrices, contenant de 0,5 à 25 mg de principe actif par kilo de poids corporel.

0067769

European Patent Office

# EUROPEAN SEARCH REPORT

Application number

EP 82 40 1058

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | MONATSHEFTE FÜR CHEMIE, vol. 109, no. 5, mai 1978, pages 1099-1113, Vienne (AT); W.FLEISCHHACKER et al.: "Basische Tetrahydro-dibenzofuran-Derivate mit einem Abstand von drei C-Atomen zwischen dem Stickstoff und dem quartären C-Atom". *Pages 1102-1103* ----- | 1,9 | C 07 D 307/91<br>A 61 K 31/34 |
| | | | TECHNICAL FIELDS SEARCHED (Int. Cl. 3) |
| | | | C 07 D 307/00 |

The present search report has been drawn up for all claims

| Place of search | Date of completion of the search | Examiner |
|---|---|---|
| THE HAGUE | 16-08-1982 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82